# EUROPEAN PATENT APPLICATION

(11) **EP 4 755 315 A1**
(43) Date of publication of application: **10.06.2026**
(21) Application number: 25218769.5
(22) Date of filing: 26.11.2025
(51) Int. Cl.: A61B 8/12, A61B 8/00

(54) **BIOPSY NEEDLE GUIDING ADAPTER AND ULTRASOUND PROBE**

(30) Priority: 05.12.2024 JP 2024212121
(71) Applicant: FUJIFILM Corporation, Tokyo 106-8620 (JP)
(72) Inventor: MIYAMOTO, Nobutaka, Tokyo, 106-8620 (JP); ISHIDA, Koichi, Tokyo, 106-8620 (JP)
(74) Representative: Meissner Bolte Partnerschaft mbB

(57) **Abstract**

Attachment of a biopsy needle guiding adapter to an ultrasound probe can be easily performed outside an abdominal cavity, and an attachment state of the biopsy needle guiding adapter in the abdominal cavity can be easily checked. Alternatively, smooth insertion and removal of the ultrasound probe from a guide tube passing through an abdominal wall can be performed.

A puncture adapter is attachably and detachably mounted on a head part of a probe outside an abdominal cavity. The puncture adapter has a body part that covers a through-hole guiding a biopsy needle, an insertion part that is inserted into the through-hole, and a needle guide hole that communicates with the body part and the insertion part and that guides the biopsy needle inserted from a needle entrance portion in the body part to a needle entrance portion in the insertion part.

## Description

### BACKGROUND OF THE INVENTION

### 1. Field of the Invention

The present invention relates to a biopsy needle guiding adapter and an ultrasound probe.

### 2. Description of the Related Art

An ultrasound diagnostic apparatus acquires biological information on a subject by transmitting ultrasound waves into the subject using an ultrasound probe and receiving reflected waves. The acquired biological information is displayed as an ultrasound image representing a state of the subject. For example, in a case where the ultrasound diagnostic apparatus is used in laparoscopic surgery using a laparoscope, the ultrasound probe is inserted into an abdominal cavity through a trocar disposed in a body wall of the subject.

In the laparoscopic surgery, puncture is performed in some cases. A practitioner inserts a biopsy needle into the abdominal cavity and observes a laparoscopic image to check a position of the biopsy needle. Then, the practitioner causes a distal end of the biopsy needle to reach a target part and then performs collection of a tissue of the target part, injection of a drug into the target part, and the like.

In order to assist the practitioner in puncture, a needle guide for guiding the biopsy needle is attached to the ultrasound probe in some cases. In this case, the needle guide is gripped with forceps, and the biopsy needle is passed through the needle guide.

However, since the position of the needle guide may fluctuate in a case where the ultrasound probe is moved or rotationally moved, or attachment to the ultrasound probe may fail in a case where the needle guide is replaced in the abdominal cavity, a puncture adapter attachable to the ultrasound probe has been proposed (for example, JP2013-233261A).

### SUMMARY OF THE INVENTION

The biopsy needle guiding adapter can be attached to the ultrasound probe outside a body cavity, but it is desirable that the biopsy needle guiding adapter is easily attached to the ultrasound probe. Further, it is preferable to adopt a structure that can easily check whether or not the biopsy needle guiding adapter is detached outside the body cavity or a state where the biopsy needle guiding adapter is attached.

An object of the present disclosure is to easily attach a biopsy needle guiding adapter to an ultrasound probe outside a body cavity and to easily check an attachment state of the biopsy needle guiding adapter in the body cavity. Alternatively, an object of the present disclosure is to perform smooth insertion and removal of the ultrasound probe from a guide tube that passes through a body wall.

According to an aspect of the present disclosure, there is provided a biopsy needle guiding adapter attachably and detachably mounted on an ultrasound probe that is inserted into a body cavity from an internal passage of a guide tube passing through a body wall and that has a distal end part in which a transducer array and a through-hole for guiding a biopsy needle are disposed, and comprising an adapter body and a pair of clip mechanisms that has an elastic member in at least a part, that extends from the adapter body in opposite directions, and that is curved along an outer surface of the ultrasound probe, in which the adapter body has a plate-shaped body part that is disposed to cover a needle entrance portion of the through-hole in a state of being mounted on the ultrasound probe, an insertion part that is inserted into the through-hole, and a needle guide hole that communicates with the body part and the insertion part and that guides the biopsy needle inserted from the body part, and as the ultrasound probe is sandwiched between the clip mechanisms in a case where the biopsy needle guiding adapter is mounted on the ultrasound probe, the biopsy needle guiding adapter is fixed to the ultrasound probe.

In addition, the body part may be formed such that thicknesses of both end parts in a direction of being inserted and removed into and from the guide tube are reduced in a state of being mounted on the ultrasound probe.

In addition, the needle guide hole may be formed such that a needle entrance portion of the needle guide hole is easily visually discriminated from surroundings thereof in a case where an inside of the body cavity is irradiated with light.

In addition, the needle guide hole may be formed in a funnel shape in which a diameter of a needle entrance portion is larger than an inner diameter of the needle guide hole.

In addition, an outer shape of the insertion part may be formed according to a shape of the through-hole.

According to another aspect of the present disclosure, there is provided an ultrasound probe in which a recess according to a shape of the body part included in the biopsy needle guiding adapter is formed in a portion that touches the body part.

In addition, a flag that guides mounting of the biopsy needle guiding adapter may be attached to an outer surface corresponding to a mounting position of the biopsy needle guiding adapter.

In addition, in a state where the biopsy needle guiding adapter is not mounted, puncture at any angle may be possible within a predetermined angular range, and in a state where the biopsy needle guiding adapter is mounted, puncture at a predetermined fixed angle may be possible.

With the present disclosure, the biopsy needle guiding adapter can be easily attached to the ultrasound probe outside the body cavity, and an attachment state of the biopsy needle guiding adapter in the body cavity can be easily checked. Alternatively, with the present disclosure, the insertion and removal of the ultrasound probe from the guide tube passing through the body wall are smoothly performed.

### BRIEF DESCRIPTION OF THE DRAWINGS

FIG. 1 is a block diagram showing a schematic configuration of an ultrasound diagnostic apparatus in the present embodiment.
FIG. 2 is an enlarged perspective view showing an example of a head part of a probe in the present embodiment.
FIG. 3 is a perspective view of a head part of the probe in the present embodiment.
FIG. 4A is a front view of the head part of the probe in the present embodiment.
FIG. 4B is a plan view of the head part of the probe in the present embodiment.
FIG. 4C is a right side view of the head part of the probe in the present embodiment.
FIG. 4D is a bottom view of the head part of the probe in the present embodiment.
FIG. 4E is a cross-sectional view of the head part shown in FIG. 4A taken along line A-A' as viewed from a left side surface direction.
FIG. 5 is a perspective view of a puncture adapter in the present embodiment.
FIG. 6A is a front view of the puncture adapter in the present embodiment.
FIG. 6B is a plan view of the puncture adapter in the present embodiment.
FIG. 6C is a right side view of the puncture adapter in the present embodiment.
FIG. 6D is a bottom view of the puncture adapter in the present embodiment.
FIG. 6E is a cross-sectional view of the puncture adapter shown in FIG. 6A taken along line C-C' as viewed from the left side surface direction.
FIG. 7A is a front view of the head part of the probe in a state where the puncture adapter is mounted in the present embodiment.
FIG. 7B is a plan view of the head part of the probe in a state where the puncture adapter is mounted in the present embodiment.
FIG. 7C is a right side view of the head part of the probe in a state where the puncture adapter is mounted in the present embodiment.
FIG. 7D is a bottom view of the head part of the probe in a state where the puncture adapter is mounted in the present embodiment.
FIG. 7E is a cross-sectional view of the probe shown in FIG. 7A taken along line D-D' as viewed from the left side surface direction.
FIG. 8 is a view showing a method of mounting the puncture adapter on the head part of the probe in the present embodiment.

### DESCRIPTION OF THE PREFERRED EMBODIMENTS

Hereinafter, an embodiment according to the present disclosure will be described with reference to the drawings.

### Configuration of Ultrasound Diagnostic Apparatus

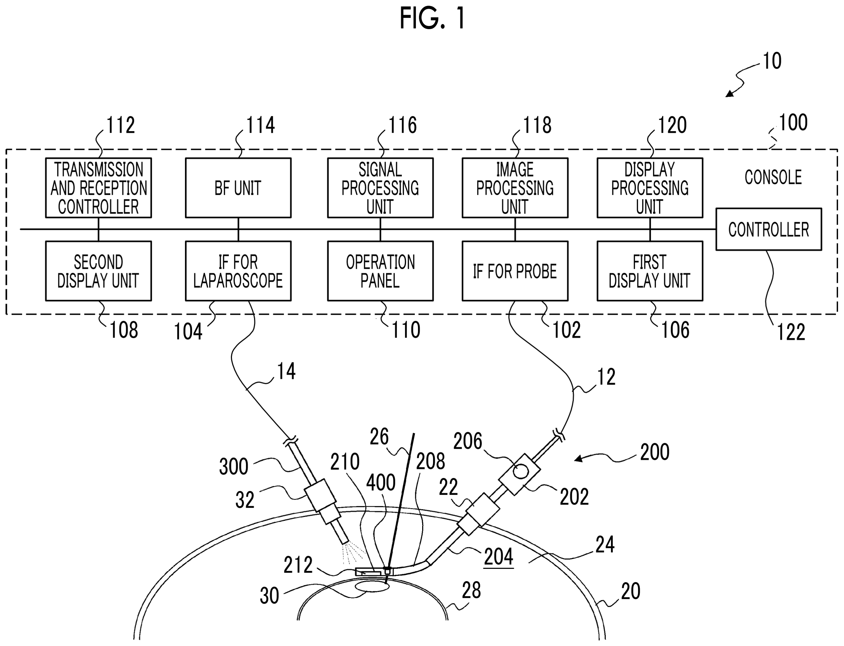

FIG. 1 is a block diagram showing a schematic configuration of an ultrasound diagnostic apparatus 10 in the present embodiment. The ultrasound diagnostic apparatus 10 in the present embodiment has an apparatus main body 100, a probe 200, and a laparoscope 300. The ultrasound diagnostic apparatus 10 can also be referred to as an ultrasound diagnostic system. The ultrasound diagnostic apparatus 10 has a function of executing ultrasound diagnosis using the probe 200 and the laparoscope 300.

The apparatus main body 100 is also called a "console" and has interfaces (IFs) 102 and 104 that connect the probe 200 and the laparoscope 300 to each other, and a first display unit 106, a second display unit 108, and an operation panel 110 as user interfaces. The first display unit 106 displays an ultrasound image generated based on an ultrasound signal received by the probe 200. The second display unit 108 displays a captured image from the laparoscope 300. Each of the display units 106 and 108 is a device that displays an image and is configured with, for example, a liquid crystal panel, an organic EL panel, or the like. The operation panel 110 is a device operated by an operator (hereinafter, also referred to as a "user") such as a practitioner in order to control input and display of a parameter or the like in ultrasound diagnosis.

The console 100 exerts an ultrasound diagnosis processing function and has a transmission and reception controller 112, a beam forming (BF) unit 114, a signal processing unit 116, an image processing unit 118, a display processing unit 120, and a controller 122.

The transmission and reception controller 112 controls transmission and reception of ultrasound waves caused by each vibration element in the probe 200. This control includes, for example, supply of an electrical transmission signal to each vibration element, amplification of an electrical reception signal from each vibration element, and the like. In the supply of the transmission signal, the transmission and reception controller 112 controls a supply timing of the transmission signal to each vibration element to form a transmission beam of the ultrasound waves.

The beam forming unit 114 performs phasing addition processing on the reception signal from each of the vibration elements in the probe 200. A reception beam is formed by the phasing addition processing. The beam forming unit 114 outputs echo data obtained along the reception beam as a result of the phasing addition processing. In addition, in a case of performing transmission beam forming, the beam forming unit 114 generates a plurality of transmission signals for the transmission beam forming.

The signal processing unit 116 performs various types of signal processing, such as gain correction processing, logarithmic amplification processing, envelope detection processing, and filter processing, on echo data output by the beam forming unit 114.

The image processing unit 118 has a coordinate transformation function and an interpolation function and forms a display frame, that is, an ultrasound image based on a plurality of pieces of beam data output from the signal processing unit 116. The beam data from the signal processing unit 116 is data in a coordinate system of beam scanning, and is configured with a plurality of data points along a direction of a beam corresponding to the beam data. The image processing unit 118 transforms, for example, a signal value of each of the data points of the beam data into a display coordinate system, that is, an ultrasound image coordinate system (generally, an orthogonal coordinate system represented by a set of an x coordinate and a y coordinate). In addition, the image processing unit 118 interpolates a value of a pixel having no value from values of surrounding pixels. The image processing unit 118 forms an ultrasound image, such as a B mode tomographic image, through such coordinate transformation and such interpolation.

The display processing unit 120 forms display screen data by combining an image, text, or the like indicating various types of information on an ultrasound image formed by the image processing unit 118. For example, information to be combined with the ultrasound image includes an ROI indicating a display range of various types of display modes such as a color Doppler mode, a sample volume of a pulse Doppler mode, a line indicating a beam in which the sample volume is positioned, and the like. Then, the display processing unit 120 displays the display screen data formed as described above on the first display unit 106.

The controller 122 controls execution of ultrasound diagnosis processing by controlling an operation of each of components included in the console 100.

Each of the components 102 to 122 in the console 100 is realized by a cooperative operation between a computer mounted on the console 100 and a program that is operated by a CPU mounted on the computer.

The probe 200 is connected to the ultrasound diagnostic apparatus 10 via a cable 12. The probe 200 in the present embodiment is an intracavitary insertion type ultrasound probe. The term "abdominal cavity" is an internal space of a person or the like and is a portion surrounded by an abdominal wall below a diaphragm, more specifically, a space that is surrounded by the abdominal wall and that has a digestive organ such as a stomach and an intestine inside. The term "body cavity" refers to a gap between a body wall and a digestive tract and mainly refers to a thoracic cavity, a pericardial cavity, and the abdominal cavity. In the present embodiment, the term "abdominal cavity" and the term "body cavity" are used synonymously.

The probe 200 mainly comprises a grip part 202 for the operator to grip and an insertion part 204 that extends from the grip part 202 and that is to be inserted into the abdominal cavity. The probe 200 may include the cable 12 described above as a component. An operation part 206 for the operator to operate is provided in the grip part 202. A trocar (guide tube) 22 that passes through an abdomen (hereinafter, referred to as an "abdominal wall") 20 of the body wall for the probe 200 is attached to the abdominal wall 20, and the insertion part 204 of the probe 200 is inserted into an abdominal cavity 24 from an internal passage of the trocar 22. In a case where an inner diameter of the trocar 22 is approximately 12 mm, an outer diameter of the insertion part 204 of the probe 200 needs to be smaller than the inner diameter of the trocar 22 and is, for example, approximately 10 mm.

The insertion part 204 has a bending part 208 that bends up, down, left, or right in response to an operation of the operation part 206 and a distal end part (hereinafter, referred to as a "head part") 210 on a distal end side extending from the bending part 208. A transducer array 212 is disposed in the head part 210 in a longitudinal direction. The transducer array 212 corresponds to "each vibration element" described above. The ultrasound diagnostic apparatus 10 performs ultrasound diagnosis inside the body cavity by causing the transducer array 212 to emit ultrasound waves. The operator bends the bending part 208 by operating the operation part 206, thereby securing a diagnosis visual field. A through-hole for guiding a biopsy needle is provided on a bending part 208 side of the head part 210. A biopsy needle guiding cutout that guides the biopsy needle may be provided on the distal end side of the head part 210.

Then, a biopsy needle guiding adapter (hereinafter, simply referred to as a "puncture adapter") 400, which is featured in the present embodiment, is attached at a position where the through-hole is disposed. A specific structure of the puncture adapter 400 and a structure of the head part 210 of the probe 200 to which the puncture adapter 400 is attached will be described later. A biopsy needle 26 is inserted from the abdominal wall 20. A distal end portion of the biopsy needle 26 passes through a needle guide hole provided in the puncture adapter 400 and reaches a target part, for example, a tumor or the like 30 of an organ 28.

In addition, a trocar 32 for inserting the laparoscope 300 into the abdominal cavity 24 is attached to the abdominal wall 20. A light source (not shown) is provided at a distal end portion of the laparoscope 300, and the laparoscope 300 images an irradiation range in the light source, for example, a range including the head part 210 of the probe 200 by being operated by the user.

The configuration of the ultrasound diagnostic apparatus 10 and a relationship between a human body to be subjected to ultrasound diagnosis and the ultrasound diagnostic apparatus 10 have been described above with reference to FIG. 1. The ultrasound diagnostic apparatus 10 in the present embodiment may have basically the same hardware configuration as in the related art. In the present embodiment, the puncture adapter 400 described below and a shape or the like of a portion of the probe 200 to which the puncture adapter 400 is attached have characteristics as necessary. Hereinafter, the structure of the puncture adapter 400 and the structure of the head part 210 of the probe 200 to which the puncture adapter 400 is attached will be described.

### Structure of Probe 200

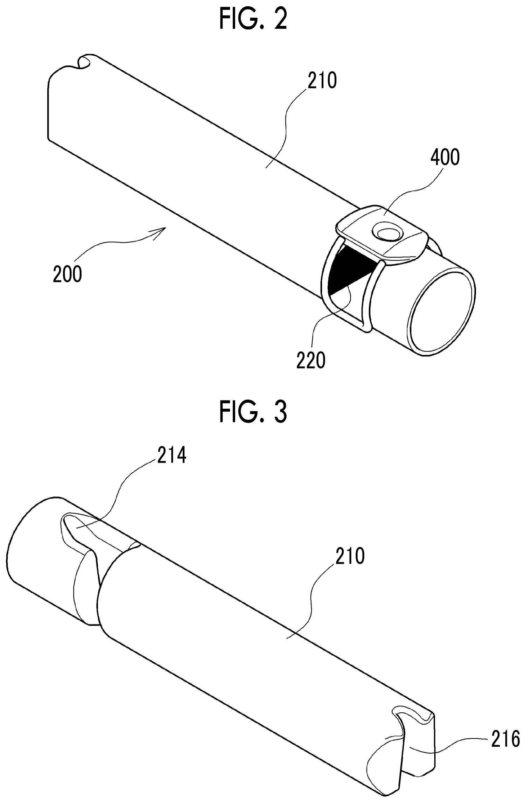

FIG. 2 is an enlarged perspective view showing an example of the head part 210 of the probe 200 shown in FIG. 1. As shown in FIG. 2, the puncture adapter 400 can be mounted on the head part 210. First, a configuration of the head part 210 in a state where the puncture adapter 400 is not mounted will be described with reference to FIGS. 1, 3, and 4A to 4E.

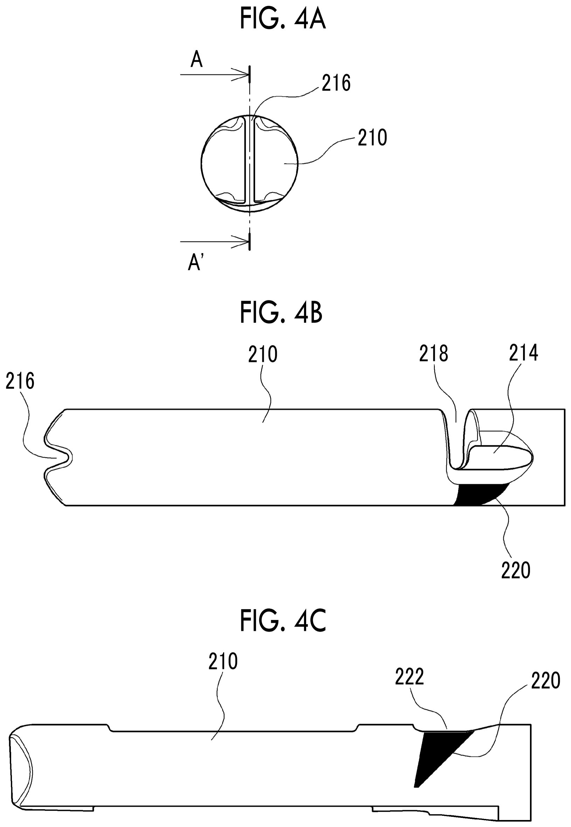

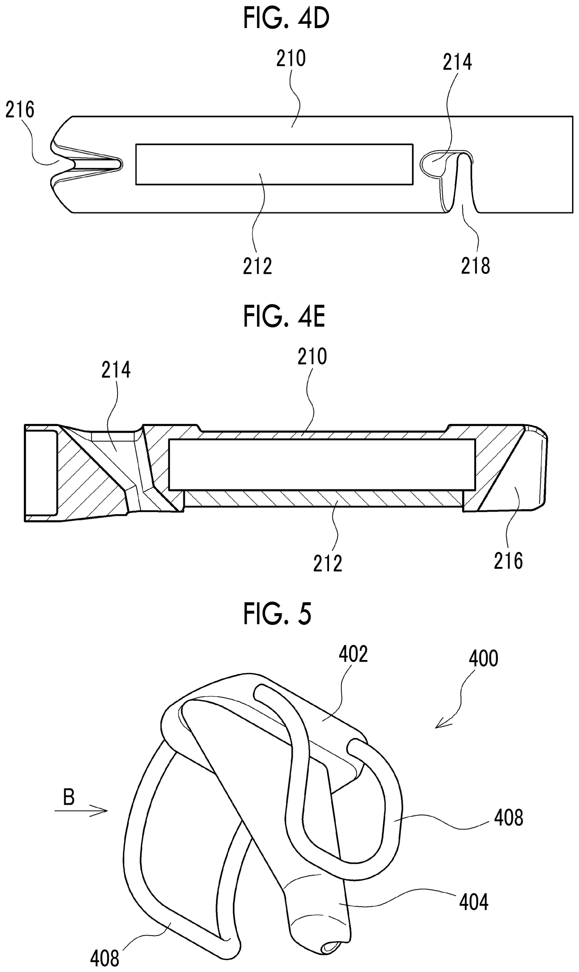

FIG. 3 is a perspective view of the head part 210 of the probe 200 used in the present embodiment. FIG. 2 is a perspective view in which a right side surface of the head part 210 is viewed from a direction of the bending part 208, while FIG. 3 is a perspective view in which a left side surface of the head part 210 is viewed from a direction of the distal end of the head part 210. In addition, FIG. 4A is a front view of the head part 210. In FIG. 4A, a surface in a case of being viewed from a distal end direction of the head part 210 is a front surface. In addition, FIG. 4B is a plan view of the head part 210. FIG. 4C is a right side view of the head part 210. FIG. 4D is a bottom view of the head part 210. FIG. 4E is a cross-sectional view of the head part 210 shown in FIG. 4A taken along line A-A' as viewed from a left side surface direction. Although components such as a circuit for transmitting and receiving an ultrasound signal may be incorporated inside the head part 210, the components are not shown in FIG. 4E.

The transducer array 212 and a through-hole 214 for guiding the biopsy needle are disposed in the head part 210, that is, a distal end part of the probe 200. In the probe 200 in the present embodiment, the through-hole 214 is provided on a bending part side of the transducer array 212. The through-hole 214 may be disposed on the distal end side of the transducer array 212. A biopsy needle guiding cutout 216 is formed in the distal end of the head part 210 in the present embodiment. By increasing a cutout amount of the biopsy needle guiding cutout 216 from an upper surface toward a lower surface of the head part 210, the biopsy needle guiding cutout 216 can guide a distal end of the biopsy needle 26 to enter below the transducer array 212.

A cross-sectional shape of a needle entrance portion of the through-hole 214 is an inverted triangular shape. That is, the through-hole 214 has a tapered shape from the needle entrance portion toward a needle exit portion. Accordingly, the distal end of the biopsy needle inserted into the through-hole 214 can be guided to enter below the transducer array 212. In a state where the puncture adapter 400 is not mounted on the probe 200, the through-hole 214 has such a shape, so that the puncture can be performed within a range of a predetermined angle. In the following description, the puncture in which the biopsy needle is guided at any angle within a predetermined angular range will be referred to as "area puncture".

As shown in FIG. 4B, in the probe 200 in the present embodiment, a cutout 218 is provided only on a left side surface side of at least one side surface of the through-hole 214. By providing the cutout 218, the user can remove the probe 200 from the biopsy needle 26 in a state where the biopsy needle 26 is inserted into the tumor or the like 30.

A mark 220 having an inverted triangular shape is attached to the head part 210 on the other side surface (in the case of the present embodiment, the right side surface) on which the cutout 218 is not provided. The mark 220 is a flag for guiding the mounting of the puncture adapter 400. That is, the mark 220 is printed in an inverted triangular shape of the through-hole 214 at a position of an outer surface corresponding to the through-hole 214. Accordingly, even in a case where the position of the through-hole 214, more specifically, the needle entrance portion of the through-hole 214 is not visible on the second display unit 108 although the right side surface is visible, the user can know the position and the size of the needle entrance portion of the through-hole 214 from the mark 220. That is, the user can insert the biopsy needle 26 into the through-hole 214 with the mark 220 as reference even in a case where the needle entrance portion of the through-hole 214 is not visible.

A recess 222 is formed in the needle entrance portion of the through-hole 214 in the upper surface of the head part 210. The puncture adapter 400 is mounted to touch a portion of the recess 222. Accordingly, a body part of the puncture adapter 400 is disposed to cover the needle entrance portion of the through-hole 214 in a portion having the recess 222. An increase in a protruding amount of the body part of the puncture adapter 400 from an outer surface of the probe 200 can be suppressed by the recess 222.

In a case where the puncture adapter 400 is mounted on the probe 200 in which the recess 222 is not formed, the recess may be formed by processing the outer surface of the probe 200, that is, a position where the puncture adapter 400 is to be mounted.

### Structure of Puncture Adapter 400

Next, a structure of the puncture adapter 400 will be described with reference to FIGS. 1, 5, and 6A to 6E.

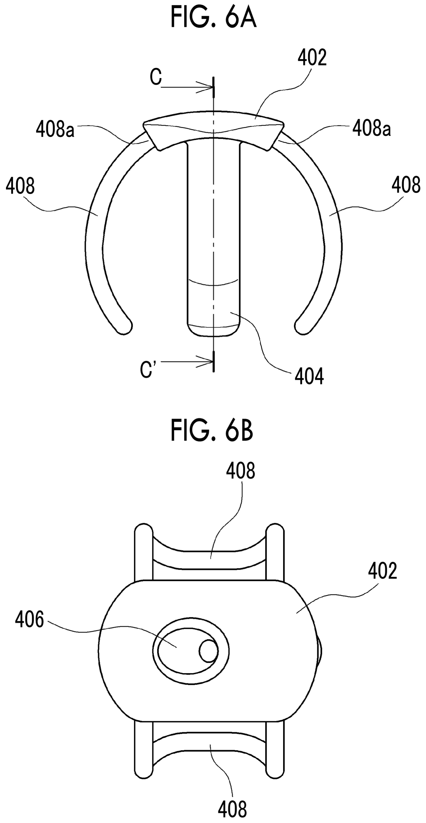

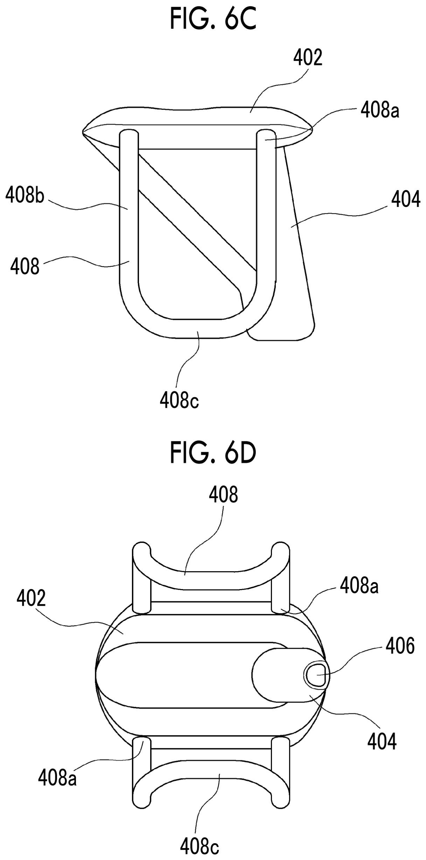

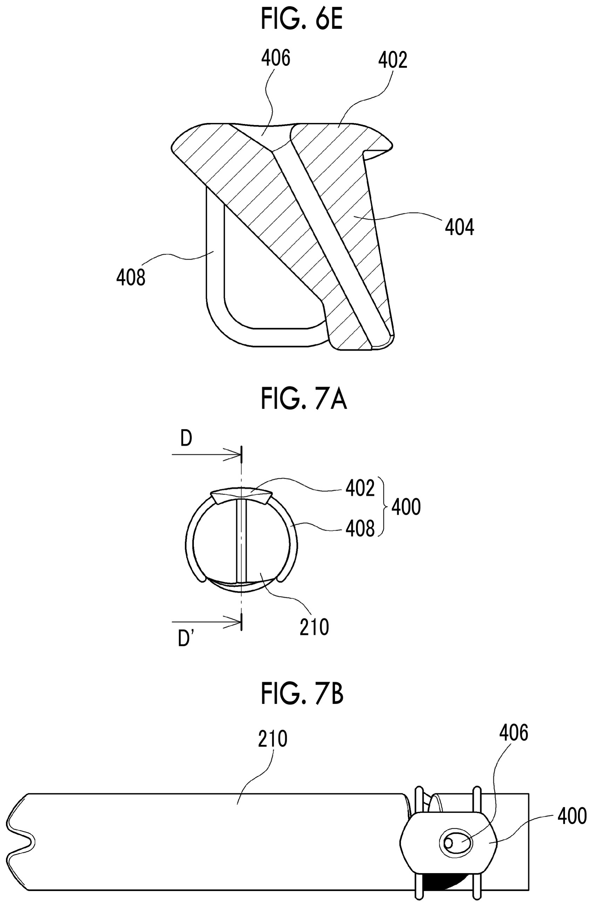

FIG. 5 is a perspective view of the puncture adapter 400 in the present embodiment. The puncture adapter 400 shown in FIG. 5 is a perspective view in an arrow B direction, that is, in a case of being viewed from the bending part 208 side in a case of being mounted on the probe 200. FIG. 6A is a front view of the puncture adapter 400. In FIG. 6A, the front surface is a surface as viewed from the bending part 208 side in a case where the puncture adapter 400 is mounted on the head part 210 of the probe 200. In addition, FIG. 6B is a plan view of the puncture adapter 400. In FIGS. 6B to 6E, a right side of the drawing is shown as a distal end part side of the head part 210, and a left side of the drawing is shown as the bending part 208 side. FIG. 6C is a right side view of the puncture adapter 400. Since the puncture adapter 400 has a line-symmetrical shape, the left side view is omitted. FIG. 6D is a bottom view of the puncture adapter 400. FIG. 6E is a cross-sectional view as viewed from a left side surface direction in a case where the puncture adapter 400 shown in FIG. 6A is taken along line C-C'.

The structure of the puncture adapter 400 can be roughly divided into an adapter body and a clip mechanism. The adapter body has a body part 402 and an insertion part 404 and further has a needle guide hole 406 that communicates with the body part 402 and the insertion part 404.

The body part 402 is formed in a thin plate shape and is disposed to cover a needle entrance portion of the through-hole 214 of the probe 200 in a case of being mounted on the probe 200. The body part 402 is formed in a thin plate shape. That is, the body part 402 has a shape with which smooth insertion and removal from the trocar 22 can be performed in a state of being mounted on the probe 200. In the present embodiment, the body part 402 is further formed such that the thicknesses of both end parts of the probe 200 in an insertion and removal direction from the trocar 22 are reduced. The body part 402 is formed in a so-called streamlined shape. Accordingly, the body part 402 is not caught in the trocar 22 or is less likely to be caught in the trocar 22, so that the probe 200 is easily inserted into and removed from the trocar 22. As described above, since the inner diameter of the trocar 22 is approximately 12 mm and the outer diameter of the insertion part 204 of the probe 200 is approximately 10 mm, the outer diameter of the insertion part 204 of the probe 200 including a top portion of the body part 402 is within 12 mm in a state where the puncture adapter 400 is mounted on the head part 210. That is, the amount of protrusion of the puncture adapter 400 from the head part 210 is suppressed to be within 2 mm. In the present embodiment, since the recess 222 is provided in the head part 210, a protruding amount from an outer periphery of the insertion part 204 can be reduced.

In a case where the puncture adapter 400 is mounted on the head part 210, the insertion part 404 is inserted into the through-hole 214 formed in the probe 200. The insertion part 404 may be formed according to the shape of the through-hole 214 formed in the probe 200. In the case of the present embodiment, the insertion part 404 is formed in an inverted triangular shape in accordance with the shape of the through-hole 214, which is an inverted triangular shape.

The needle guide hole 406 linearly penetrates from the needle entrance portion provided in the upper surface of the body part 402 to the needle exit portion provided in the tapered distal end part of the insertion part 404, and guides the biopsy needle 26 inserted from the body part 402 to the target part (in the case of the present embodiment, the tumor or the like 30). As shown in FIG. 6E, the needle guide hole 406 has a funnel shape in which the diameter of the needle entrance portion of the needle guide hole 406 is larger than the inner diameter of the needle guide hole 406. By forming the needle guide hole 406 in a funnel shape and forming the needle entrance portion of the needle guide hole 406 to be wide, the user can easily insert the biopsy needle 26 into the needle guide hole 406. Even in a case where the distal end of the biopsy needle 26 reaches the funnel-shaped portion, the distal end is guided to the needle guide hole 406 by sliding on the funnel-shaped inclined surface.

In addition, the needle entrance portion of the needle guide hole 406, that is, the surroundings of the hole may be further formed to be subjected to processing such as chamfered processing and curved surface processing. The needle entrance portion of the needle guide hole 406 is irradiated with light from the light source of the laparoscope 300. However, in a case where the needle entrance portion is irradiated with light in the abdominal cavity 24, the needle entrance portion of the processed needle guide hole 406 reflects the light in a direction different from the direction of the surroundings. Accordingly, the user easily visually discriminates the needle entrance portion of the needle guide hole 406 from the surroundings thereof.

Meanwhile, in the probe 200 in a state where the puncture adapter 400 is mounted, the puncture is possible at a fixed angle. In the following description, the puncture in which the biopsy needle is guided at a predetermined fixed angle will be referred to as "line puncture".

According to the present embodiment, the area puncture can be performed with the probe 200 in a state where the puncture adapter 400 is not mounted. Since the puncture adapter 400 in the present embodiment has a shape that enables being mounted on the through-hole 214 in which the area puncture is possible, the line puncture can be performed with the probe 200 in a state where the puncture adapter 400 is mounted. That is, the user can perform the area puncture or the line puncture using the same probe 200 by selecting whether or not to mount the puncture adapter 400 to the probe 200 outside the abdominal cavity.

One needle guide hole 406 is provided in the puncture adapter 400 in the present embodiment, but a plurality of needle guide holes 406 may be provided. For example, the plurality of needle guide holes 406 in which at least one of the angle or the inner diameter is different from the others may be provided. In addition, a plurality of puncture adapters 400 having the needle guide holes 406 in which at least one of the angle or the inner diameter is different from the others may be provided.

On the other hand, the clip mechanism is formed of a pair of clips 408 that extends from a side surface of the body part 402 in opposite directions and that is curved along the outer surface of the probe 200. The pair of clips 408 has an elastic member such as spring steel. For example, a root portion of each of the clips 408, that is, a portion (hereinafter, referred to as a "bonding part 408a") that is bonded to the body part 402 is formed of spring steel or the like. The bonding part 408a of each of the clips 408 is bonded to the body part 402 by laser welding, brazing, soldering, or the like.

In order to make it difficult for the clips 408 of the puncture adapter 400 to be caught in a case where the insertion part 204 of the probe 200 is inserted into and removed from the trocar 22, it is suitable that the curved shapes of the clips 408 are formed to coincide with the outer surface of the probe 200 at a mounting position as shown in FIG. 7A.

In the present embodiment, a metal such as stainless steel having a circular cross section is curved to form the clips 408 in a U-shape. Then, as described above, both end parts of the clips 408 are configured to be bonded to the body part 402. More specifically, each of the clips 408 is formed of the bonding part 408a that is bonded to the body part 402, two leg parts 408b that extend from the bonding part 408a in a direction away from the body part 402, and a coupling part 408c that couples the two leg parts 408b. Since the clips 408 do not have a corner part, the abdominal wall 20 can be hardly damaged in a case where the probe 200 is inserted into and removed from the trocar 22. Even in a case where the probe 200 is provided with the cutout 218 as in the present embodiment, the puncture adapter 400 can be reliably fixed to the head part 210 by three of the four leg parts 408b.

It is evident that the shapes of the clips 408 are not limited thereto, and it is sufficient that the clips 408 are visible in a state of being mounted on the probe 200. For example, the clips 408 may be formed of a curved flat leaf spring or the like.

In the present embodiment, the puncture adapter 400 is formed of a metal such as stainless steel. This is because there is a possibility in which the biopsy needle 26 becomes stuck in a case where the puncture adapter 400 is formed of a material such as a resin, unlike in a case where the puncture adapter 400 is made of a hard metal. However, the puncture adapter 400 may be formed of a material such as a resin insofar as the function as the puncture adapter 400 can be exhibited.

### Engagement Relationship Between Puncture Adapter 400 and Head Part 210

Next, a configuration in a state where the puncture adapter 400 is mounted on the head part 210 of the probe 200 will be described with reference to FIGS. 1, 2, and 7A to 7E.

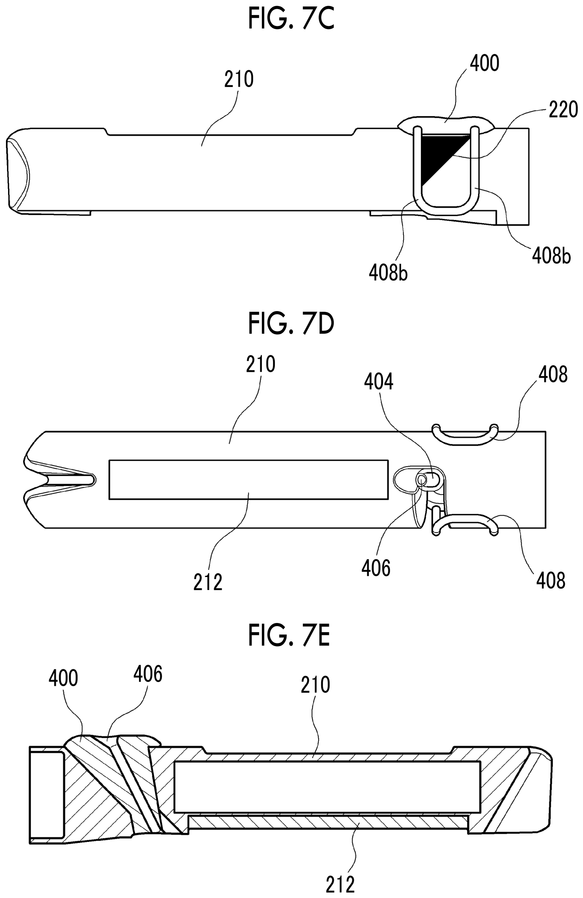

FIG. 7A is a front view of the head part 210. In FIG. 7A, a surface in a case where the head part 210 is viewed from the distal end direction of the probe 200 is a front surface. In addition, FIG. 7B is a plan view of the head part 210. FIG. 7C is a right side view of the head part 210. FIG. 7D is a bottom view of the head part 210. FIG. 7E is a cross-sectional view of the head part 210 shown in FIG. 7A taken along line D-D' as viewed from the left side surface direction. FIGS. 7A to 7E basically show a state where the puncture adapter 400 shown in FIG. 5 is mounted on the head part 210 shown in FIGS. 4A to 4E. Although components such as a circuit for transmitting and receiving an ultrasound signal may be incorporated inside the head part 210, the components are not shown in FIG. 7E. In addition, a perspective view of a state where the puncture adapter 400 is mounted on the head part 210 of the probe 200 is shown in FIG. 2.

In a state where the puncture adapter 400 is mounted on the head part 210 of the probe 200, the body part 402 of the puncture adapter 400 covers the through-hole 214 of the head part 210, for example, as shown in FIG. 7B. In addition, by forming the clips 408 in a U-shape, for example, as shown in FIG. 7C, even in a case where the puncture adapter 400 is mounted on the probe 200, the mark 220 positioned on the right side surface of the head part 210 can be viewed from between the leg parts 408b of the clips 408.

### Method of Attaching and Detaching Puncture Adapter 400 to and from Probe 200

Next, a method of attaching and detaching the puncture adapter 400 to and from the probe 200 will be described.

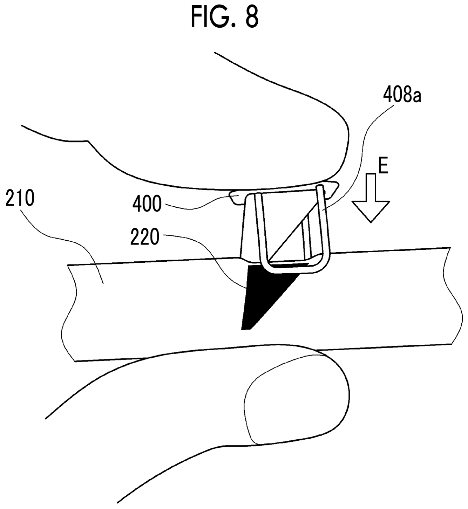

The puncture adapter 400 in the present embodiment is attached to the probe 200 outside the abdominal cavity. As shown in FIG. 8, the user aligns the puncture adapter 400 with the position of the through-hole 214 of the probe 200. In a case where the through-hole 214 is not visible, the alignment is performed directly above the mark 220 attached to the probe 200. Then, the user brings the fingers holding the puncture adapter 400 and the probe 200 close to each other. For example, the user applies a force in an arrow E direction shown in FIG. 8 to the finger that supports the puncture adapter 400. Due to this pressing force, the force acts in an opening direction on the bonding part 408a of the puncture adapter 400 formed of an elastic member, and the puncture adapter 400 is pushed until the body part 402 of the puncture adapter 400 touches the recess 222 of the probe 200. In a case where the coupling part 408c of each clip 408 is pushed into the probe 200, the coupling part 408c of each clip 408 slides on the outer surface of the probe 200. In a case where the body part 402 touches the recess 222 of the probe 200, the force is not applied in the opening direction on the bonding part 408a. Then, the expanded clip 408 returns to a state before the mounting. As a result, the puncture adapter 400 is mounted on the head part 210 of the probe 200 as shown in FIG. 2.

In addition, in a case where the puncture adapter 400 is pushed from above, a distal end of the insertion part 404 slides down an inclined surface of the through-hole 214. For this reason, the user can easily mount the puncture adapter 400 at a predetermined position of the head part 210 only by pushing the puncture adapter 400 from above.

The puncture adapter 400 is inserted into the abdominal cavity 24 together with the insertion part 204 of the probe 200 in a state of being mounted on the head part 210. The puncture adapter 400 functions as a guide that guides the biopsy needle 26 in the abdominal cavity 24.

The puncture adapter 400 in the present embodiment is reliably fixed to the probe 200 by a gripping force of spring steel forming a part of the clip 408. However, since the puncture adapter 400 has an attachable and detachable structure, there is a possibility in which the puncture adapter 400 is detached from the probe 200 for some reason. In the present embodiment, since the clip 408 that fixes the puncture adapter 400 to the probe 200 appears on the outer surface of the probe 200, a mounting state of the puncture adapter 400 in the abdominal cavity can be easily checked visually by the captured image from the laparoscope 300.

The puncture adapter 400 is taken out of the abdominal cavity 24 via the trocar 22 in a state of being mounted on the head part 210 of the probe 200. The user removes the puncture adapter 400 from the probe 200 by, for example, pushing up the coupling part 408c of the clip 408 of the puncture adapter 400 from below or pulling the body part 402 of the puncture adapter 400 from above.

According to the present embodiment, the puncture adapter 400 that is attachable and detachable to and from the probe 200 can be provided outside the abdominal cavity 24. In addition, as a shape that suppresses an increase in the protruding amount of the puncture adapter 400 from an outer periphery of the probe 200 is adopted, the insertion and removal of the probe 200 via the trocar 22 can be smoothly performed. Further, an attachment state of the puncture adapter 400 to the probe 200 in the abdominal cavity 24 can be easily checked.

### Explanation of References

10: ultrasound diagnostic apparatus
12: cable
20: abdominal wall
22, 32: trocar
24: abdominal cavity
26: biopsy needle
28: organ
30: tumor or the like
100: console
106: first display unit
108: second display unit
110: operation panel
112: transmission and reception controller
114: beam forming (BF) unit
116: signal processing unit
118: image processing unit
120: display processing unit
122: controller
200: probe
202: grip part
204: insertion part
206: operation part
208: bending part
210: head part
212: transducer array
214: through-hole
216: biopsy needle guiding cutout
218: cutout
220: mark
222: recess
300: laparoscope
400: puncture adapter
402: body part
404: insertion part
406: needle guide hole
408: clip
408a: bonding part
408b: leg part
408c: coupling part

## Claims

1. A biopsy needle guiding adapter attachably and detachably mounted on an ultrasound probe that is inserted into a body cavity from an internal passage of a guide tube passing through a body wall and that has a distal end part in which a transducer array and a through-hole for guiding a biopsy needle are disposed, the biopsy needle guiding adapter comprising:
an adapter body; and
a pair of clip mechanisms that has an elastic member in at least a part, that extends from the adapter body in opposite directions, and that is curved along an outer surface of the ultrasound probe,
wherein the adapter body has
a plate-shaped body part that is disposed to cover a needle entrance portion of the through-hole in a state of being mounted on the ultrasound probe,
an insertion part that is inserted into the through-hole, and
a needle guide hole that communicates with the body part and the insertion part and that guides the biopsy needle inserted from the body part, and
as the ultrasound probe is sandwiched between the clip mechanisms in a case where the biopsy needle guiding adapter is mounted on the ultrasound probe, the biopsy needle guiding adapter is fixed to the ultrasound probe.

2. The biopsy needle guiding adapter according to claim 1,
wherein the body part is formed such that thicknesses of both end parts in a direction of being inserted and removed into and from the guide tube are reduced in a state of being mounted on the ultrasound probe.

3. The biopsy needle guiding adapter according to claim 1 or 2,
wherein the needle guide hole is formed such that a needle entrance portion of the needle guide hole is easily visually discriminated from surroundings thereof in a case where an inside of the body cavity is irradiated with light.

4. The biopsy needle guiding adapter according to any one of claims 1 to 3,
wherein the needle guide hole is formed in a funnel shape in which a diameter of a needle entrance portion is larger than an inner diameter of the needle guide hole.

5. The biopsy needle guiding adapter according to any one of claims 1 to 4,
wherein an outer shape of the insertion part is formed according to a shape of the through-hole.

6. An ultrasound probe in which a recess according to a shape of the body part included in the biopsy needle guiding adapter according to any one of claims 1 to 5 is formed in a portion that touches the body part.

7. The ultrasound probe according to claim 6,
wherein a flag that guides mounting of the biopsy needle guiding adapter is attached to an outer surface corresponding to a mounting position of the biopsy needle guiding adapter.

8. The ultrasound probe according to claim 6 or 7,
wherein in a state where the biopsy needle guiding adapter is not mounted, puncture at any angle is possible within a predetermined angular range, and
in a state where the biopsy needle guiding adapter is mounted, puncture at a predetermined fixed angle is possible.
